(19) Europäisches Patentamt
European Patent Office
Office européen des brevets

(11) **EP 4 335 427 B1**

(12) **EUROPEAN PATENT SPECIFICATION**

(45) Date of publication and mention
of the grant of the patent:
**29.10.2025 Bulletin 2025/44**

(21) Application number: **22820490.5**

(22) Date of filing: **03.06.2022**

(51) International Patent Classification (IPC):
**A61H 1/02** *(2006.01)*      **A61H 3/00** *(2006.01)*
**A61B 5/00** *(2006.01)*

(52) Cooperative Patent Classification (CPC):
**A61H 3/00; A61B 5/00; A61H 1/0244;**
A61H 2201/1207; A61H 2201/165;
A61H 2201/5007; A61H 2201/5061;
A61H 2201/5069; A61H 2230/625

(86) International application number:
**PCT/KR2022/007921**

(87) International publication number:
**WO 2022/260362 (15.12.2022 Gazette 2022/50)**

(54) **NON-THERAPEUTIC METHOD AND APPARATUS FOR OUTPUTTING TORQUE TO PROVIDE FORCE TO USER**

NICHT-THERAPEUTISCHES VERFAHREN UND VORRICHTUNG ZUR AUSGABE EINES DREHMOMENTS ZUR BEREITSTELLUNG VON KRAFT AN EINEN BENUTZER

PROCÉDÉ NON THÉRAPEUTIQUE ET APPAREIL POUR DÉLIVRER UN COUPLE POUR FOURNIR UNE FORCE À UN UTILISATEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.06.2021  KR 20210073650**
**16.03.2022  KR 20220032678**

(43) Date of publication of application:
**13.03.2024  Bulletin 2024/11**

(73) Proprietor: **Samsung Electronics Co., Ltd.**
**Suwon-si, Gyeonggi-do 16677 (KR)**

(72) Inventors:
• **LIM, Bokman**
**Suwon-si Gyeonggi-do 16677 (KR)**

• **SEO, Keehong**
**Suwon-si Gyeonggi-do 16677 (KR)**
• **LEE, Jusuk**
**Suwon-si Gyeonggi-do 16677 (KR)**

(74) Representative: **Gulde & Partner**
**Patent- und Rechtsanwaltskanzlei mbB**
**Berliner Freiheit 2**
**10785 Berlin (DE)**

(56) References cited:
EP-A1- 3 037 081        JP-A- 2010 063 813
JP-A- 2015 164 451      JP-A- 2017 099 798
KR-A- 20090 111 343    KR-B1- 102 186 859
US-A1- 2012 215 140    US-A1- 2015 366 738
US-B2- 10 350 129        US-B2- 9 610 209

**Description**

BACKGROUND

Technical Field

The disclosure relates to a non-therapeutic method and wearable device for outputting a torque to provide a force to a user, and more particularly, to a non-therapeutic method and apparatus for providing an assistive force and a resistive force to the user wearing a wearable device.

Background Art

With the onset of aging societies, a growing number of people experience inconvenience and pain in walking from weakened muscular strength or joint problems from aging, and there is growing interest in walking assisting devices that enable the elderly with weakened muscular strength or patients with muscular joint discomfort to walk with ease. Methods and devices supporting people when walking are disclosed in EP 3 037 081 A1, US 10 350 129 B2, US 2012 / 215140 A1, US 9 610 209 B2 and US 2015 / 366738 A1. Document EP 3 037 081 A1 discloses a method for an assisting torque setting apparatus that involves obtaining a first angle of a first joint and a second angle of a second joint of a user's legs, calculating evaluation values for gait indices such as gait symmetry, stride length, and foot clearance based on these angles, and setting a stable assisting torque by applying weights to an initial torque corresponding to the indices. The method further includes setting a basic assisting torque corresponding to the user's gait motion and controlling a motor driver to output a final assisting torque, which is determined by combining the stable and basic torques, to assist the user's walking.

SUMMARY OF THE INVENTION

The present invention is defined in the independent claims, with optional features being defined in the dependent claims.

SUMMARY OF THE DISCLOSURE

Technical Goals

Additional aspects will be set forth in part in the description which follows and, in part, will be apparent from the description, or may be learned by practice of the presented embodiments.

According to an aspect of the invention, a non-therapeutic method, performed by a wearable device, of outputting a torque includes obtaining a first angle of a first joint of a first leg of a user and a second angle of a second joint of a second leg of the user, obtaining a first adjustment angle, based on an offset angle set for the first angle and the first joint, obtaining a first state factor associated with the first angle and the second angle, based on the first adjustment angle and the second angle, obtaining a first value of a parameter for adjusting at least one of a magnitude, a direction, and timing of a torque to be output, obtaining a first torque value, based on the first state factor and the first value of the parameter, and controlling a motor driver of the wearable device to output the first torque value. A maximum value of the first torque value increases as the offset angle increases.

The offset angle may be set by the user.

The non-therapeutic method may include obtaining the offset angle, based on a first angular trajectory of the first angle of the first joint and a second angular trajectory of the second angle of the second joint.

Obtaining the offset angle may include determining a target asymmetry degree, based on the first angular trajectory and the second angular trajectory.

The first state factor may be associated with a distance between the first leg and the second leg.

Obtaining the first state factor may include determining an initial state factor, based on the first adjustment angle and the second angle. The first state factor may be determined based on a previous state factor and the initial state factor.

According to the invention, a flexion interval of the first joint increases as the offset angle increases.

Further according to the invention, a flexion interval of the second joint decreases as the offset angle increases.

The parameter may include at least one of a gain and a delay associated with the first state factor.

A first value of the gain and a first value of the delay may be set differently based on a target operation mode among one or more operation modes of the wearable device.

The one or more operation modes may include an assistive mode for outputting the first torque value in a first direction that is the same as a second direction in which the first joint moves and a resistive mode for outputting the first torque value in a third direction opposite to the second direction in which the first joint moves.

The first value of the parameter may be determined differently based on the offset angle.

According to another aspect of the invention, a non-transitory computer-readable storage medium stores instructions that, when executed by a processor, cause the processor to obtain a first angle of a first joint of a first leg of a user and a second angle of a second joint of a second leg of the user, obtain a first adjustment angle, based on an offset angle set for the first angle and the first j oint, obtain a first state factor associated with the first angle and the second angle, based on the first adjustment angle and the second angle, obtain a first value of a parameter for adjusting at least one of a magnitude, a direction, and timing of a torque to be output, obtain a first torque value, based on the first state factor and the first value of the parameter, and control a motor driver of the wearable device to output the first torque value. A maximum value of the first torque value increases as the offset angle increases.

According to yet another aspect of the invention, a wearable device configured to provide a force to a user includes at least one sensor configured to measure an angle of a joint of the user, a motor driver circuit, and a motor electrically connected to the motor driver circuit; and a processor configured to obtain, by the at least one sensor, a first angle of a first joint of a first leg of the user and a second angle of a second joint of a second leg of the user, obtain a first adjustment angle, based on an offset angle set for the first angle and the first joint, obtain a first state factor associated with the first angle and the second angle, based on the first adjustment angle and the second angle, obtain a first value of a parameter for adjusting at least one of a magnitude, a direction, and timing of a torque to be output, obtain a first torque value, based on the first state factor and the first value of the parameter, and control the motor driver circuit of the wearable device to output the first torque value. A maximum value of the first torque value may increase as the offset angle increases. The force provided to the user may correspond the output first torque value.

The offset angle may be set by the user.

The processor may be further configured to obtain the offset angle, based on a first angular trajectory of the first angle of the first joint and a second angular trajectory of the second angle of the second joint.

The processor may be further configured to obtain a target asymmetry degree, based on the first angular trajectory and the second angular trajectory and determine the offset angle based on the target asymmetry degree.

The processor may be further configured to obtain an initial state factor, based on the first adjustment angle and the second angle and obtain the first state factor, based on a previous state factor and the initial state factor.

The first value of the parameter may be set differently based on a target operation mode among one or more operation modes of the wearable device.

The one or more operation modes may include an assistive mode for outputting the first torque value in a first direction that is the same as a second direction in which the first joint moves and a resistive mode for outputting the first torque value in a third direction opposite to the second direction in which the first joint moves.

Brief Description of Drawings

The above and other aspects, features, and advantages of certain embodiments of the present disclosure will be more apparent from the following description taken in conjunction with the accompanying drawings, in which:

FIGS. 1A, 1B, 1C and 1D are diagrams each illustrating a wearable device according to embodiments;
FIG. 2 is a diagram illustrating a wearable device communicating with an electronic device according to an embodiment;
FIGS. 3 and 4 are diagrams each illustrating a wearable device according to another example embodiment;
FIG. 5 is a flowchart illustrating a method of outputting a torque according to an example embodiment;
FIG. 6 is a diagram illustrating a symmetric gait according to an example embodiment;
FIG. 7 is a flowchart illustrating a method of determining an offset angle, based on an asymmetry degree according to an example embodiment;
FIGS. 8A and 8B are diagrams each illustrating an asymmetric gait according to an example embodiment;
FIG. 9 is a diagram illustrating trajectories of torque values output when applying different offset angles to a user according to an example embodiment;
FIG. 10 is a diagram illustrating a trajectory of a hip joint angle of a user with an asymmetric gait according to an example embodiment;
FIG. 11 is a diagram illustrating a trajectory of a torque value output when applying an offset angle to a user with a symmetric gait according to an example embodiment;
FIG. 12 is a flowchart illustrating a method of determining a first parameter, based on an operation mode of a wearable device according to an example embodiment; and
FIG. 13 is a diagram illustrating a trajectory of a torque value output in a resistive mode according to an example embodiment.

Best Mode for Carrying Out the Invention

Hereinafter, various example embodiments of the present disclosure will be described with reference to the accompanying drawings. However, this is not intended to limit the present disclosure to specific example embodiments, and it should be understood that various modifications, equivalents, and/or alternatives of the embodiments of the present disclosure are included.

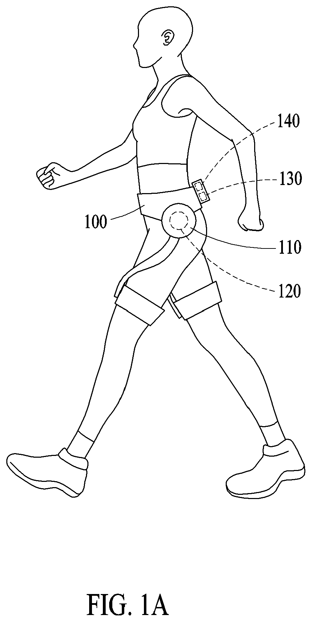

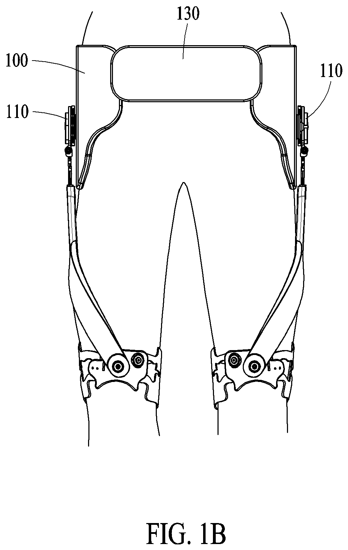

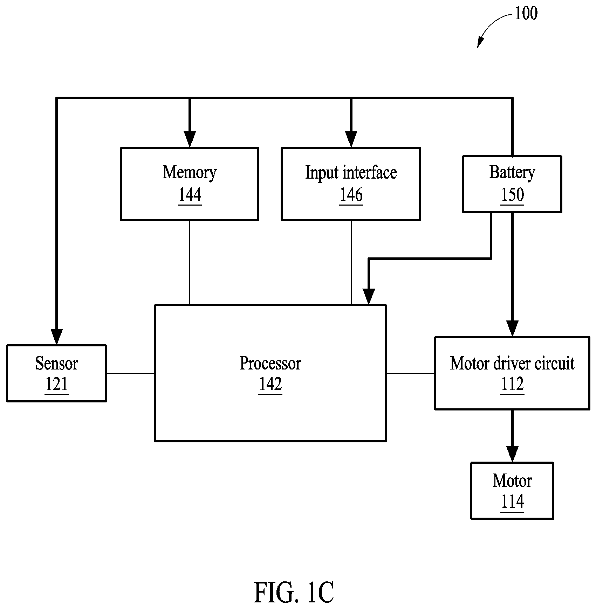

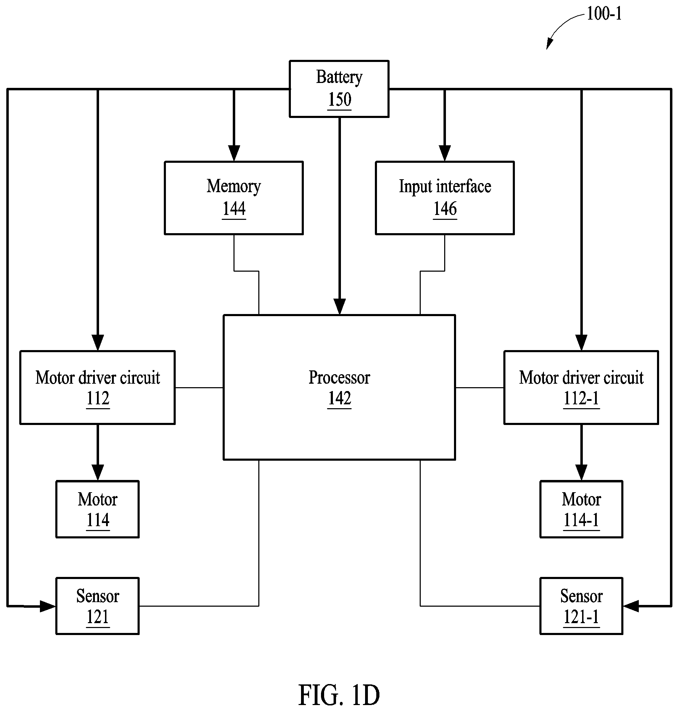

FIGS. 1A, 1B, 1C and 1D are diagrams each illustrating a wearable device according to embodiments of the disclosure.

Referring to FIGS. 1A to 1D, a wearable device 100 may be disposed on a user to assist walking. The wearable device 100 may be a device for assisting the user in walking. The wearable device 100 may also be an exercise device for providing an exercise function to the user by providing a resistive force. The resistive force provided to the user, for example, may be an active force applied to the user, such as a force output by a device, such as a motor, and as another example, may be a force resisting motion of the user, such as a frictional force, which is not the active force applied to the user. In other words, the resistive force may be expressed as an exercise load.

Although the wearable device 100 illustrated in FIGS. 1A to 1D is a hip type, a type of the wearable device of the present disclosure may not be limited to a hip type, and the wearable device may be a type supporting an entire lower limb or a portion of the lower limb. In addition, the wearable device may be any one of a form supporting a portion of the lower limb, a form supporting the knee or ankle, and a form supporting the entire body.

Although example embodiments described with reference to FIGS. 1A-1D may be applied to a hip type, example embodiments may not be limited to a hip type and be applied to various types of wearable devices.

The wearable device 100 may include a driver 110, a sensor 120, an inertial measurement unit (IMU) 130, a controller 140, a battery 150, and a communication module. For example, the IMU 130 and the controller 140 may be disposed in a main frame of the wearable device 100. As another example, the IMU 130 and the controller 140 may be included in a housing, which is formed (or attached to) outside the main frame.

The driver 110 may include a motor 114 and a motor driver circuit 112 for driving the motor 114. The sensor 120 may include at least one sensor 121. The controller 140 may include a processor 142, a memory 144, and an input interface 146. Although the wearable device 100 is illustrated as including one sensor 121, one motor driver circuit 112, and one motor 114 in FIG. 1C, this is only an example. That is, as another example, a wearable device 100-1, as illustrated in FIG. 1D, may include a plurality of sensors 121 and 121-1, a plurality of motor driver circuits 112 and 112-1, and a plurality of motors 114 and 114-1. Also, the wearable device 100 may include a plurality of processors according to implementation. The number of motor driver circuits, the number of motors, or the number of processors may vary based on the body part on which the wearable device 100 is worn.

The following descriptions on the sensor 121, the motor driver circuit 112, and the motor 114 may also be applied to the sensor 121-1, the motor driver circuit 112-1, and the motor 114-1 illustrated in FIG. 1D.

The driver 110 may drive a hip joint of the user. The driver 110 may be disposed on, for example, the right hip and/or the left hip of the user. The driver 110 may be disposed additionally on the knee and ankle of the user. The driver 110 may include the motor 114 for generating a rotational torque and the motor driver circuit 112 for driving the motor 114.

The sensor 120 may measure an angle of the hip joint of the user while walking. Information on the angle of the hip joint sensed by the sensor 120 may include an angle of the right hip joint, an angle of the left hip joint, a difference between the angles of the right and left hip joints, and a hip joint motion direction. For example, the sensor 121 may be disposed in the driver 110. The sensor 120 may additionally measure a knee angle and an ankle angle of the user according to a position of the sensor 121. The sensor 121 may be an encoder. Information on an angle of a joint measured by the sensor 120 may be transmitted to the controller 140.

The sensor 120 may include a potentiometer. The potentiometer may sense an R-axis joint angle and an L-axis joint angle, and an R-axis joint angular velocity and an L-axis joint angular velocity, based on walking motion of the user. R and L axes may respectively be reference axes for the right leg and the left leg of the user. For example, the R and L axes may be set to be vertical to the ground and set such that a front side of the body of a person has a negative value and a rear side of the body of the person has a positive value.

The IMU 130 may measure acceleration information and posture information while walking. For example, the IMU 130 may sense X-axis, Y-axis, and Z-axis acceleration and an X-axis, a Y-axis, and a Z-axis angular velocity, based on walking motion of the user. The acceleration information and the posture information measured by the IMU 130 may be transmitted to the controller 140.

The wearable device 100 may detect a point on which the foot of the user lands, based on the acceleration information measured by the IMU 130.

A pressure sensor may be disposed on a sole of the user and detect the point on which the foot of the user lands.

In addition to the sensor 120 and the IMU 130 described above, the wearable device 100 may include another sensor, such as an electromyogram (EMG) sensor, for sensing a change of a quantity of motion or a bio-signal of the user, based on walking motion of the user.

The controller 140 may control the overall operation of the wearable device 100. For example, the controller 140 may receive information sensed by each of the sensor 120 and the IMU 130. The information sensed by the IMU 130 may include the acceleration information and the posture information. The information sensed by the sensor 120 may include the angle of the right hip j oint, the angle of the left hip j oint, the difference between the angles of the right and left hip joints, and the hip joint motion direction. The controller 140 may calculate the difference between the angles of the right and left hip joints, based on the angle of the right hip joint and the angle of the left hip joint. The controller 140 may generate a signal for controlling the driver 110, based on the sensed information. For example, the generated signal may be an assistive force for assisting the user in walking. As another example, the generated signal may be a resistive force for preventing the user from walking. The resistive force may be provided for the user to exercise.

According to an example embodiment, the processor 142 of the controller 140 may control the driver 110 to provide the resistive force to the user.

For example, the driver 110 may provide the resistive force to the user by applying an active force to the user through the motor 114. As another example, the driver 110 may provide the resistive force to the user by using back-drivability of the motor 114 without applying the active force to the user. When the back-drivability increases, a motor may more readily respond to an external force acting on the rotation axis, that is, the rotation axis of the motor may more readily rotate. For example, even when the same external force is applied to the rotation axis of the motor, a degree of rotation of the rotation axis of the motor may change according to a degree of back-drivability.

According to another example embodiment, the driver 110 may provide the resistive force to the user by outputting a torque in a direction that hinders motion of the user.

According to an example embodiment, the processor 142 of the controller 140 may control the driver 110 such that the driver 110 outputs a torque (or an assistive torque) for assisting the user in walking. For example, in a hip-type wearable device (e.g., the wearable device 100), the driver 110 may be disposed on each of a left hip portion and a right hip portion, and the controller 140 may output a control signal for controlling the driver 110 to generate a torque.

The driver 110 may generate a torque, based on the control signal output by the controller 140. A torque value for generating the torque may be externally set or set by the controller 140. For example, to represent a magnitude of the torque value, the controller 140 may use a magnitude of a current of a signal transmitted to the driver 110. That is, as the magnitude of the current received by the driver 110 increases, the torque value may increase. As another example, the processor 142 of the controller 140 may transmit the control signal to the motor driver circuit 112 of the driver 110, and the motor driver circuit 112 may generate a current corresponding to the control signal to control the motor 114.

The battery 150 may supply power to components of the wearable device 100. The wearable device 100 may further include a circuit (e.g., a power management integrated circuit (PMIC)) configured to convert power of the battery 150 to match an operating voltage of the components of the wearable device 100 and provide the converted power to the components of the wearable device 100. In addition, the battery 150 may or may not supply power to the motor 114, based on an operation mode of the wearable device 100.

The communication module may support the establishment of a direct (or wired) communication channel or a wireless communication channel between the wearable electronic device 100 and an external electronic device and support communication through the established communication channel. The communication module may include one or more communication processors configured to support direct (or wired) communication or wireless communication. According to an example embodiment, the communication module may include a wireless communication module (e.g., a cellular communication module, a short-range wireless communication module, or a global navigation satellite system (GNSS) communication module) or a wired communication module (e.g., a local area network (LAN) communication module or a power line communication (PLC) module). A corresponding one of these communication modules may communicate with the external electronic device via a first network (e.g., a short-range communication network, such as Bluetooth™, wireless-fidelity (Wi-Fi) direct, or infrared data association (IrDA)) or a second network (e.g., a long-range communication network, such as a legacy cellular network, a 5G network, a next-generation communication network, the Internet, or a computer network). These various types of communication modules may be implemented as a single component (e.g., a single chip), or may be implemented as multiple components (e.g., multiple chips) separate from each other.

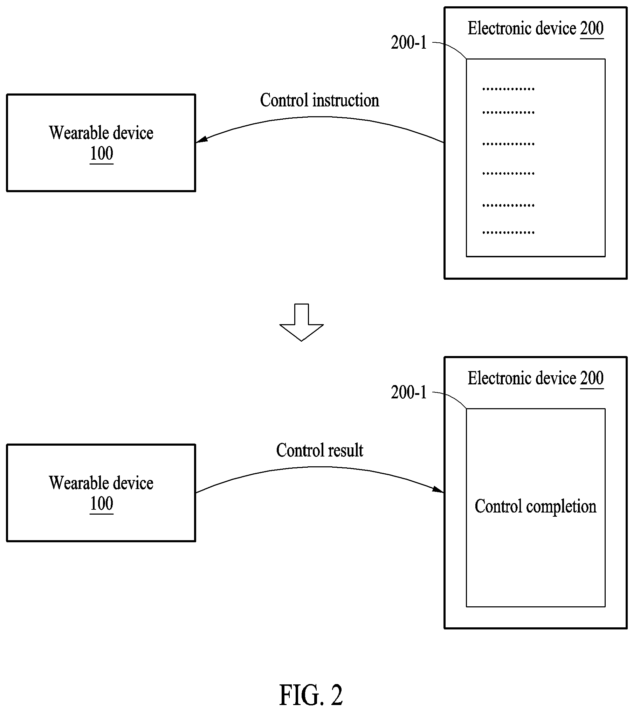

FIG. 2 is a diagram illustrating a wearable device communicating with an electronic device according to an embodiment of the disclosure.

Referring to FIG. 2, a wearable device 100 may communicate with an electronic device 200. The electronic device 200 may include, for example, a smartphone, a tablet, a smartwatch, eyeglasses, and the like, but may not be limited to the foregoing examples. For example, the electronic device 200 may be an electronic device of a user of the wearable device 100. As another example, the electronic device 200 may be an electronic device of a trainer who guides the user wearing the wearable device 100.

According to implementation, the wearable device 100 and the electronic device 200 may communicate through a server via short-range wireless communication or cellular mobile communication.

The electronic device 200 may display a user interface (UI) for controlling an operation of the wearable device 100 on a display 200-1. The UI may include, for example, at least one soft key through which the user may control the wearable device 100.

The user may input a command for controlling the operation of the wearable device 100 through the UI on the display 200-1 of the electronic device 200, and the electronic device 200 may generate a control instruction corresponding to the command and transmit the generated control instruction to the wearable device 100. The wearable device 100 may operate according to the received control instruction and transmit a control result to the electronic device 200. The electronic device 200 may display a control completion message on the display 200-1 of the electronic device 200.

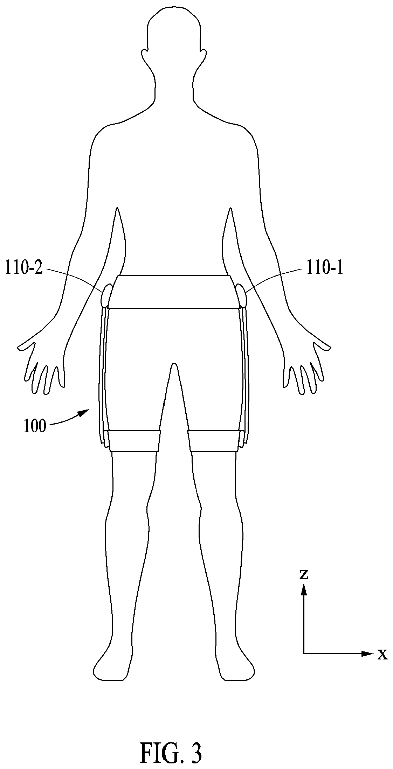

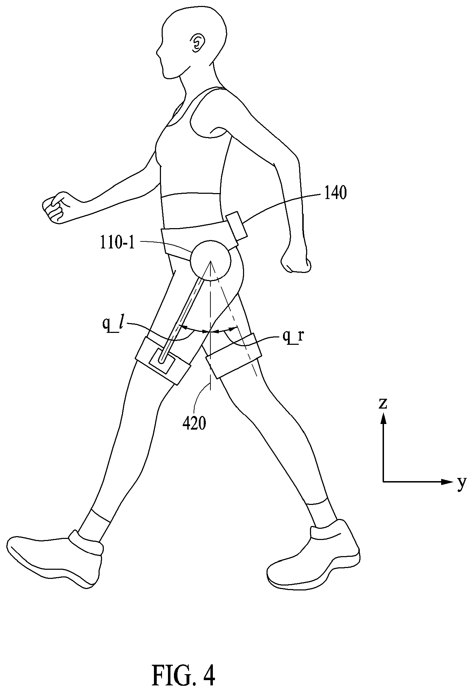

FIGS. 3 and 4 are diagrams illustrating a wearable device according to embodiments of the disclosure.

Referring to FIGS. 3 and 4, drivers 110-1 and 110-2 of the wearable device 100 of FIGS. 1A to 1D may be disposed around a hip joint of a user, and the controller 140 of the wearable device 100 may be disposed around the waist of the user. The positions of the drivers 110-1 and 110-2 and the controller 140 are not limited to the positions illustrated in FIGS. 3 and 4 as examples.

The wearable device 100 may measure (or sense) a left hip joint angle q_l and a right hip joint angle q_r of the user. For example, the wearable device 100 may measure the left hip joint angle q_l of the user through a left encoder and measure the right hip joint angle q_r of the user through a right encoder. As illustrated in FIG. 4, the left hip joint angle q_l may be a negative number because the left leg of the user is before a reference line 420, and the right hip joint angle q_r may be a positive number because the right leg of the user is behind the reference line 420. According to some embodiments, the right hip joint angle q_r may be negative when the right leg is before the reference line 420, and the left hip joint angle q_l may be positive when the left leg is behind the reference line 420.

According to an example embodiment, the wearable device 100 may determine a torque value $\tau(t)$ based on the left hip joint angle q_l, the right hip joint angle q_r, a gain $\kappa$, and a delay $\Delta t$, and control the motor driver circuit 112 of the wearable device 100 to output the determined torque value $\tau(t)$. A force provided to the user by the torque value $\tau(t)$ may be referred to herein as force feedback. For example, the wearable device 100 may determine the torque value $\tau(t)$ based on Equation (1).

$$y = sin(q\_r) - sin(q\_l) \qquad (1)$$

In Equation (1), y denotes a state factor, and q_r denotes a right hip joint angle, and q_l denotes a left hip joint angle. According to Equation (1), the state factor y may be associated with a distance between both legs. For example, y being 0 may represent a state (e.g., a crossing state) in which the distance between the legs is 0, and a maximum absolute value of y may represent a state (e.g., a landing state) in which an angle between the legs is a maximum. When q_r and q_l are measured at a time t, the state factor may be represented as y(t).

The gain $\kappa$ is a parameter representing a magnitude and a direction of an output torque. As the magnitude of the gain $\kappa$ increases, a greater torque may be output. When the gain $\kappa$ is negative, a torque acting as a resistive force may be output to the user. When the gain $\kappa$ is positive, a torque acting as an assistive force may be output to the user. The delay $\Delta t$ is a parameter associated with torque output timing. A value of the gain $\kappa$ and a value of the delay $\Delta t$ may be preset and adjusted by the user or the wearable device 100. A model for outputting a torque acting as the assistive force to the user, based on Equation (1) and parameters, such as the gain $\kappa$ and the delay $\Delta t$, may be defined as a torque output model (or a torque output algorithm). The wearable device 100, by inputting values of input parameters received through sensors to the torque output model, may determine a magnitude of a torque to be output and a delay.

According to an example embodiment, when the user performs a gait where the left and right legs of the user are asymmetric, the wearable device 100 may provide an asymmetric torque to each leg to assist the user with an asymmetric gait while walking. The wearable device 100 may provide, for example, a stronger assistive force to the leg with a shorter stride or a slower swing speed. The leg with a shorter stride or a slower swing speed hereinafter may be referred to as an affected leg or a target leg.

According to an example embodiment, when a magnitude of a torque acting on the affected leg increases, the user may experience inconvenience from a difference between respective torques to the affected leg and the unaffected leg.

The affected leg may have a shorter swing time or a shorter stride than that of the unaffected leg. According to an example embodiment, a method of adjusting timing of the torque acting on the affected leg may be applied to assist the user in walking. For example, an offset angle may be added to an actual joint angle of the affected leg to increase an output time of a torque to assist the swing of the affected leg.

By adding the offset angle to the actual joint angle of the affected leg, a value of an input parameter input to the torque output model disposed (or applied) to the wearable device 100 may be adjusted. In the foregoing method of adjusting the value of the input parameter by adding the offset angle to the actual joint angle, a torque output model for outputting a torque to assist a user with a symmetric gait while walking may also be applied to the user with an asymmetric gait. When using the actual joint angle of the affected leg as an input value for the torque output model, because of the shorter stride and the slower swing speed of the affected leg, the torque output model may provide a weak assistive force for assisting the swing of the affected leg at belated timing. However, when using a value of the offset angle added to the actual joint angle of the affected leg as the input value for the torque output model, because of a stride and a swing speed increased by the offset angle, the torque output model may provide a relatively stronger assistive force for assisting the swing of the affected leg at relatively earlier timing. As an offset is added to a joint angle of the affected leg, a relatively weaker assistive force for assisting a swing of a healthy leg may be provided at relatively later timing than when the offset is not added. Although the assistive force for assisting the swing of the healthy leg is weak, the user may not experience inconvenience.

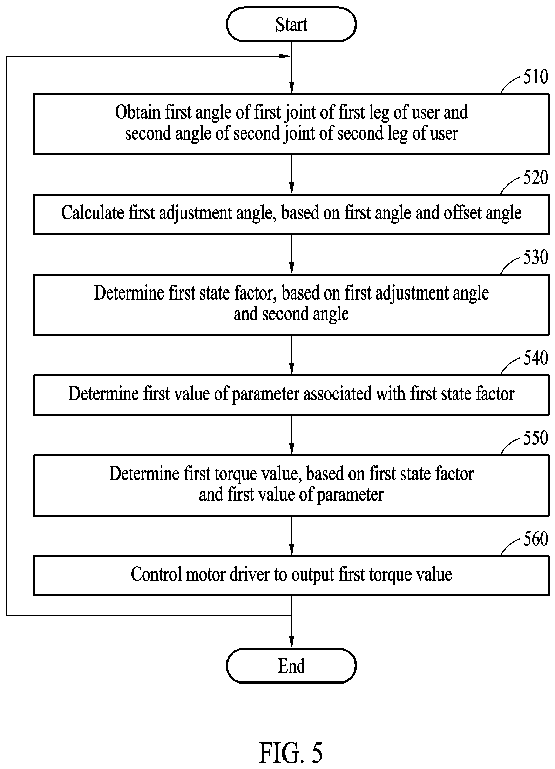

The method of outputting a torque to assist the user in walking, based on the offset angle added to the joint angle of the user, is described further below with reference to FIGS. 5 through 11.

FIG. 5 is a flowchart illustrating a method of outputting a torque according to an embodiment.

According to an example embodiment, operations 510 through 560 to be described herein with reference to FIG. 5 may be performed by the controller 140 (or the processor 142 of the controller 140) of the wearable device 100.

In operation 510, the processor 142 may obtain a first angle (e.g., q_r) of a first joint of a first leg of a user wearing the wearable device 100 and a second angle (e.g., q_1) of a second joint of a second leg of the user. For example, the first joint and the second joint may respectively be the right hip joint and the left hip joint of the user but may not be limited thereto. As another example, the first joint and the second joint may respectively be the right knee joint and the left knee joint of the user. In another example, the first joint and the second joint may respectively be the right ankle joint and the left ankle joint of the user.

According to an example embodiment, the sensor 120 (or an encoder) disposed around a joint of the user may sense an angle of the joint, and the processor 142 may obtain the sensed angle of the joint from the sensor 120. The sensed angle of the joint may be associated with a timestamp at a sensed time.

According to an example embodiment, the processor 142 may obtain the first angle and the second angle by filtering a first raw angle (e.g., q_r_raw) and a second raw angle (e.g., q_l_raw) sensed by the sensor 120. For example, the first raw angle and the second raw angle may be filtered based on a first previous angle and a second previous angle sensed at a previous time. A raw angle may mean a raw angle represented by a value sensed by the sensor 120. The raw angle may be largely different from an actual angle due to noise that may be caused during sensing. The processor 142, to decrease an impact of the noise, may obtain the first angle by filtering the first raw angle sensed at a current time, based on the first previous angle sensed at the previous time. The filtering of the raw angle may decrease a range of a change even when there is a sharp change in the raw angle, compared to a previous angle.

In operation 520, the processor 142 obtains and e.g. calculates a first adjustment angle, based on the first angle and a set offset angle (e.g., c). For example, the first angle may be an angle of a first joint of an affected leg of the user. When the first angle is q_r, and an offset angle is c, the first adjustment angle may be calculated as q_r + c. As a gait asymmetry degree increases, the offset angle may increase.

According to an example embodiment, the offset angle may be an angle set by the user. For example, the offset angle may be an angle between 0 to 25 degrees. The user may specifically input the offset angle to the wearable device 100 every time or input a signal for increasing the offset angle compared to a previous offset angle or a signal for decreasing the offset angle compared to the previous offset angle. When the user feels that a start time of a torque output to a swing of the affected leg is later than a desired time, the offset angle set for the wearable device 100 may be increased compared to a current angle. As another example, the user may input an offset level having a corresponding offset angle to the wearable device 100.

According to an example embodiment, the user may input a desired offset angle through a UI of the wearable device 100. The user may input the desired offset angle through a button or a soft button implemented through a display as the UI of the wearable device 100.

According to an example embodiment, the user may input the desired offset angle to the wearable device 100 through the electronic device 200 connected to the wearable device 100. The electronic device 200 may transmit, to the wearable device 100, information on an offset angle input by the user.

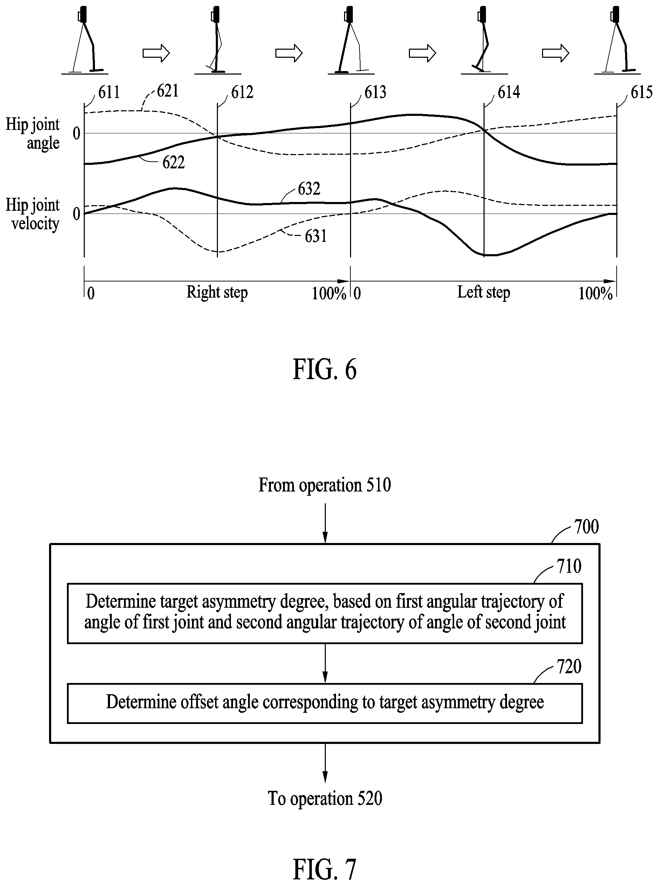

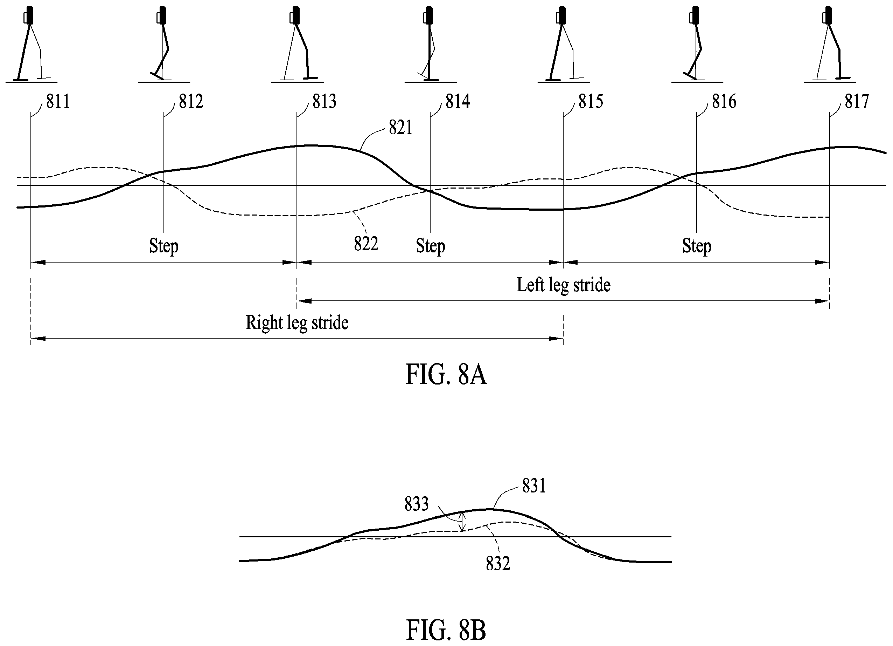

According to an example embodiment, an offset angle may be determined by the wearable device 100. For example, the wearable device 100 may determine an asymmetry degree, based on a walking state of the user, and determine the offset angle, based on the determined asymmetry degree. The determined offset angle may be an optimal offset angle that may provide an assistive force suitable for the asymmetry degree of the user. The method, performed by the wearable device 100, of determining the offset angle is described further below with reference to FIGS. 7 and 8.

Although the method of calculating the first adjustment angle is described in operation 520, besides the calculation of the first adjustment angle, a second adjustment angle may be calculated, based on the second angle and a second offset angle.

In operation 530, the processor 142 obtains and e.g. determines a first state factor associated with a distance between the first leg and the second leg, based on the first adjustment angle and the second angle.

According to an example embodiment, the first state factor may be calculated based on Equation (2).

$$y = \sin(q_{\_r} + c) - \sin(q_{\_l}) \qquad (2)$$

The description of Equation (1) may be similarly applied to the description of Equation (2).

According to an example embodiment, to decrease inconvenience felt by the user, a state factor may be filtered. For example, the processor 142, based on the first adjustment angle and the second angle, may determine an initial state factor $y_{raw}$ at a current time t, and based on a previous state factor $y^{prv}$ determined at a previous time t-1 and the initial state factor $y_{raw}$, may determine a first state factor y.

$$y = (1 - \alpha)y^{prv} + \alpha y_{raw} \qquad (3)$$

In Equation (3), $\alpha$ denotes a preset constant.

In operation 540, the processor 142 obtains and e.g. determines a first value of a parameter associated with the first state factor. The parameter may be a parameter for adjusting at least one of a magnitude, a direction, and timing of a torque to be output. For example, the parameter may include at least one of a gain and a delay associated with the state factor. When the parameter is any one of the gain and the delay, the parameter may be one dimensional (1D), and when the parameter includes the gain and the delay, the parameter may be two dimensional (2D). A first gain value and a first delay value may be preset.

According to an example embodiment, a value of the gain and a value of the delay may be adjusted for personalization. The user may adjust the value of the gain and the value of the delay to minimize inconvenience.

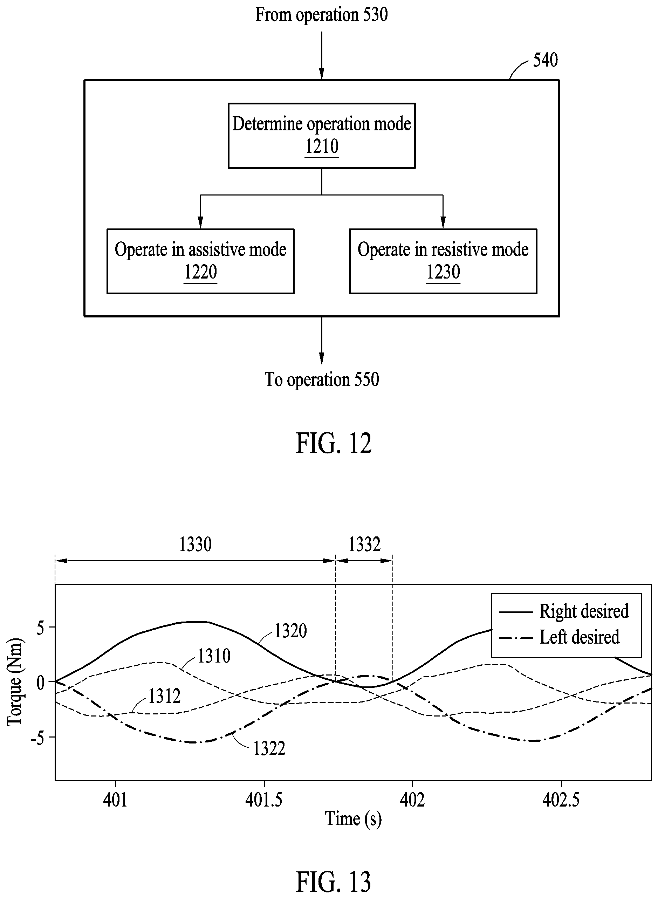

According to an example embodiment, the wearable device 100 may operate in any one operation mode among a plurality of operation modes, and the first value of the parameter may vary, based on the operation mode. For example, when the parameter includes at least one of the gain and the delay, a first value of the gain and a first value of the delay may be set differently based on a target operation mode among the operation modes of the wearable device 100. The method of determining the first value of the parameter, based on the operation mode, is described further below with reference to FIG. 12.

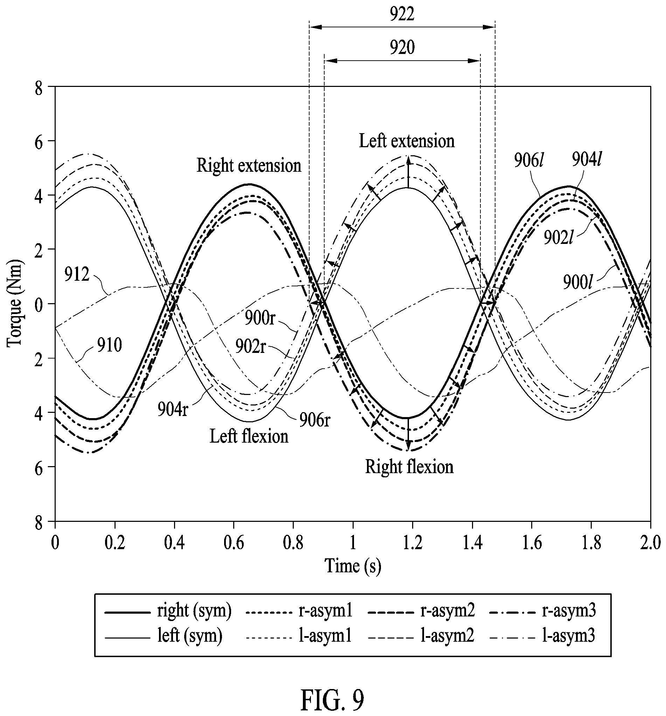

According to an example embodiment, when the set offset angle varies, the first value of the parameter may vary. For example, when the first angle of the first joint of the user and the second angle of the second joint of the user are obtained, a value of the parameter determined with a first offset angle being set and the value of the parameter determined with the second offset angle being set may vary, where the second offset angle is different from the first offset angle. As the offset angle applied to the first angle increases, a flexion interval of the first joint may increase, and a parameter value may be determined such that a torque output in the flexion interval may increase. The flexion interval will be described in detail with reference to FIG. 9.

According to an example embodiment, even when the set offset angle varies, the first value of the parameter may not vary. For example, when the first angle of the first joint of the user and the second angle of the second joint of the user are obtained, the value of the parameter determined with the first offset angle being set and the value of the parameter determined when the second offset angle different from the first offset angle being set may be the same. For example, even when the value of the parameter determined for offset angles that are different from each other is the same, as the offset angle increases, a maximum value of a torque output to the first joint may increase.

In operation 550, the processor 142, based on the first state factor and the first value of the parameter, obtains and e.g. determines a first torque value. For example, the first torque value may be calculated based on Equation (4).

$$\tau_l(t) = \kappa y(t - \Delta t)$$
$$\qquad (4)$$
$$\tau_r(t) = -\kappa y(t - \Delta t)$$

The calculated first torque value may be applied to both legs and may thus include a value for the first joint and a value for the second joint. For example, $\tau_l(t)$ denotes a value for the left hip joint, which is the second joint, and $\tau_r(t)$ denotes a value for the right hip joint, which is the first joint. $\tau_l(t)$ and $\tau_r(t)$ may be values having the same magnitude and opposite torque directions.

In operation 560, the processor 142 controls a motor driver circuit to output the first torque value. For example, the processor 142 of the wearable device 100 may provide an assistive force to the user by controlling the motor 114, based on the first torque value. The wearable device 100, by using the battery 150 of the wearable device 100, may drive the motor 114 to output an assistive torque, and the assistive torque output by the motor 114 may provide the assistive force to the user. The first torque value may be a control signal applied to the motor 114, the assistive torque may be a rotational torque output by the motor 114, based on the first torque value, and the assistive force may be a force felt by the user through the assistive torque.

FIG. 6 is a diagram illustrating a symmetric gait according to an embodiment.

The graph illustrated in FIG. 6 may represent a change of gait data according to a gait of a user. The horizontal axis may represent time and the vertical axis may represent a hip joint angle or a hip joint velocity.

At a time point 611, the right leg may stop swinging, at a time point 612, the left leg may swing and cross the right leg, at a time point 613, the left leg may stop swinging, at a time point 614, the right leg may swing and cross the left leg, and at a time point 616, the right leg may stop swinging as at the time point 611. In other words, walking motion at the time points 611 to 615 may represent a stride, walking motion at the time point 611 to 613 may represent a step of the right leg (or a swing of the left leg), and walking motion at the time points 613 through 615 may represent a step of the left leg (or a swing of the right leg). The stride may refer to a process that a position of any one leg returns to the same position through walking. A dashed line 621 may represent a left hip joint angle, a solid line 622 may represent a right hip joint angle, a dashed line 631 may represent a left hip joint velocity, and a solid line 632 may represent a right hip joint velocity.

When the user performs a symmetric gait, the left hip joint angle and the right hip joint angle may represent similar trajectories, and the left hip joint velocity and the right hip joint velocity may also represent almost similar trajectories.

According to an example embodiment, when the user performs an asymmetric gait, the left hip joint angle and the right hip joint angle may be asymmetric. For example, an interval of the left hip joint angle and an interval of the right hip joint angle may be different from each other (that is, a swing speed of each leg may be different). As another example, a maximum angle (or a minimum angle) of the left hip joint angle may be significantly different from a maximum angle (or a minimum angle) of the right hip joint angle (that is, a stride of each leg may be different).

For example, the reason why the user performs the asymmetric gait may be because of insufficient strength to swing an affected leg. In this case, when an assistive torque to assist a swing of the affected leg is output at relatively earlier timing and for a longer time than the case of an symmetric gait, the asymmetric gait may change to a more symmetric gait.

FIG. 7 is a flowchart illustrating a method of determining an offset angle, based on an asymmetry degree according to an embodiment.

According to an example embodiment, operation 700 may be further performed after operation 510 described above with reference to FIG. 5 is performed. Operation 700 may include operations 710 and 720.

In operation 710, the processor 142 may determine a target asymmetry degree, based on a first angular trajectory of an angle of a first joint and a second angular trajectory of an angle of a second joint. For example, an angular trajectory may be generated based on a timestamp of angles of a joint. The method of determining the target asymmetry degree is described further below with reference to FIG. 8.

In operation 720, the processor 142 may determine an offset angle corresponding to the target asymmetry degree. For example, as the target asymmetry degree increases, a greater offset angle may be determined. The offset angle determined by the processor 142 may be an optimal offset angle.

FIGS. 8A and 8B are diagrams each illustrating an asymmetric gait according to an embodiment.

The graph illustrated in FIG. 8A may represent a change of a hip joint angle according to a gait of a user. In the graph of FIG. 8A, the horizontal axis may represent time, and the vertical axis may represent a hip joint angle.

At a time point 811, the left leg may stop swinging, at a time point 812, the right leg may swing and cross the left leg, at a time point 813, the right leg may stop swinging, at a time point 814, the left leg may swing and cross the right leg, and at a time point 815, the left leg may stop swinging. In addition, at a time point 816, the right leg may swing and cross the left leg, and at a time point 817, the right leg may stop swinging as at the time point 813. Walking motion at the time points 811 through 815 may represent a stride of the right leg, and walking motion at the time points 813 through 817 may represent a stride of the left leg. In addition, walking motion at the time points 811 through 813 and walking motion at the time points 815 through 817 may represent a step of the left leg, and walking motion at the time points 813 through 815 may represent a step of the right leg. A solid line 821 may represent a right hip joint angle, and a dashed line 822 may represent a left hip joint angle.

According to an example embodiment, the processor 142 may estimate a similarity between a trajectory (represented as the solid line 821) of the right hip joint angle and a trajectory (represented as the dashed line 822) of the left hip joint angle, and based on the estimated similarity, determine a target asymmetry degree. For example, the target asymmetry degree may be determined to be inversely proportional to the estimated similarity.

The processor 142 may calculate a difference between a trajectory of the right hip joint angle at the time points 811 through 815 and a trajectory of the left hip joint angle at the time points 813 through 817. In this case, the trajectory of the right hip joint angle at the time points 811 through 815 and the trajectory of the left hip joint angle at the time points 813 through 817 may be one step interval.

The graph illustrated in FIG. 8B may represent a difference 833 between the trajectory (e.g., a solid line 831) of the right hip joint angle at the time points 811 through 815 and the trajectory (e.g., a dashed line 832) of the left hip joint angle at the time points 813 through 817. In the graph of FIG. 8B, the horizontal axis may represent a normalized stride time (NST), and the vertical axis may represent a hip joint angle.

According to an example embodiment, the processor 142 may determine an asymmetry degree, based on at least one of the difference 833 between the trajectories (represented respectively as the solid line 831 and the dashed line 832) and a scaling factor applied for the NST of the trajectories (represented respectively as the solid line 831 and the dashed line 832).

FIG. 9 is a diagram illustrating trajectories of torque values output when applying different offset angles to a user according to an embodiment.

According to an example embodiment, a first angular trajectory 910 of a first joint (e.g., the right hip joint) of the user and a second angular trajectory 912 of a second joint (e.g., the left hip joint) of the user may be symmetric. When the user performs a symmetric gait, and an offset angle to an angle of a certain joint (e.g., the first joint) is not applied, a torque trajectory 900r of the first joint of the user and a torque trajectory 900l of the second joint of the user may be calculated. Because the torque trajectory 900r and the torque trajectory 900l are similar, the user may not experience any particular inconvenience.

According to an example embodiment, when the offset angle is applied to a sensed angle of the first joint, torque trajectories output respectively for the first joint and the second joint may vary compared to the symmetric gait.

For example, when a first offset angle is applied to an angle of the first joint, a torque trajectory 902r may be represented for the first joint, and a torque trajectory 902l may be represented for the second joint. When a second offset angle that is greater than the first offset angle is applied to the angle of the first joint, a torque trajectory 904r may be represented for the first joint, and a torque trajectory 904l may be represented for the second joint. When a third offset angle that is greater than the second offset angle is applied to the angle of the first joint, a torque trajectory 906r may be represented for the first joint, and a torque trajectory 906l may be represented for the second joint. As the offset angle increases, in a flexion interval of the first joint to which the offset angle is applied, a relatively increased torque value may be output. Conversely, as the offset angle increases, in an extension interval of the first joint to which the offset angle is applied, a relatively decreased torque value may be output.

As the offset angle applied to the first joint increases, the flexion interval of the first joint may increase. In other words, as the offset angle applied to the first joint increases, the flexion interval of the first joint may increase. A length 922 of the flexion interval of the first joint when the third offset angle is applied is longer than a length 920 of the flexion interval of the first joint when the offset angle is not applied. That is, as the offset angle applied to the first joint increases, the wearable device 100 may assist a first leg with more force and longer in the flexion interval of the first joint.

Conversely, as the offset angle applied to the first joint increases, a length of a flexion interval of the second joint decreases, and a time for which a torque is output to the second joint may thus decrease.

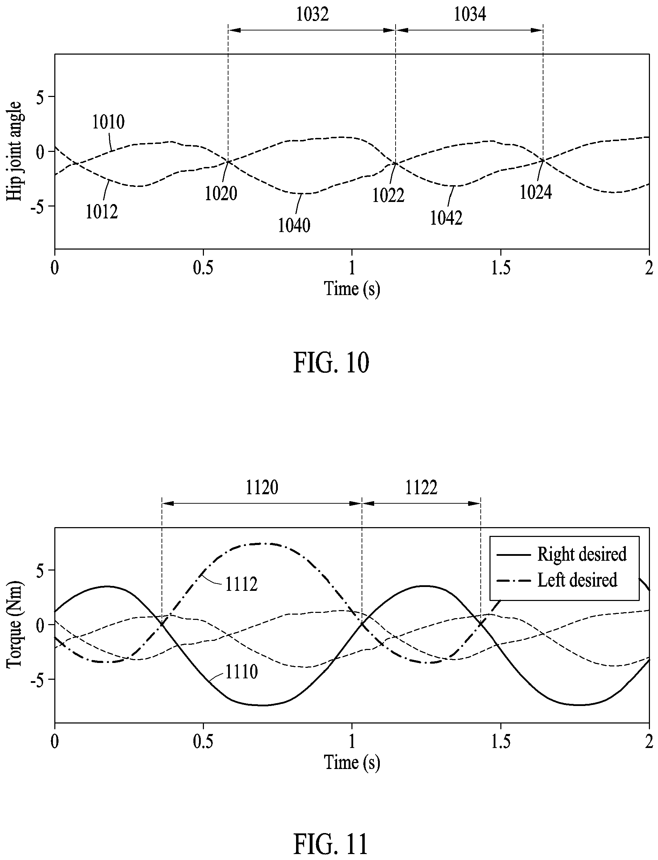

FIG. 10 is a diagram illustrating a trajectory of a hip joint angle of a user with an asymmetric gait according to an embodiment.

According to an example embodiment, gait asymmetry of the user may be represented through a first angular trajectory 1010 of a first joint and a second angular trajectory 1012 of a second joint.

The gait asymmetry, for example, may be represented as a large difference between a first time interval 1032 from a first time at which the first joint and the second joint cross one another to a second time at which the first joint and the second joint cross one another again and a second time interval 1034 from the second time to a third time at which the first joint and the second joint cross one another yet again. In the example illustrated in FIG. 10, the second interval 1034 of flexion of the second joint is shorter than the first interval 1032 of flexion of the first joint, and the first joint may thus be a joint of an affected leg.

As another example, the gait asymmetry may be represented as a large difference between a minimum angle 1040 of the first joint and a minimum angle 1042 of the second joint. In the example illustrated in FIG. 10, an absolute value of the minimum angle 1042 of the second joint is less than an absolute value of the minimum angle 1040 of the first joint, and the first joint may thus be the joint of the affected leg.

FIG. 11 is a diagram illustrating a trajectory of a torque value output when applying an offset angle to a user with a symmetric gait according to an embodiment.

According to an example embodiment, the user may perform an asymmetric gait in the first angular trajectory 1010 and the second angular trajectory 1012 described above with reference to FIG. 10. The wearable device 100 may output a torque to assist an affected leg of the user performing such an asymmetric gait. The wearable device 100, for example, may assist the affected leg by outputting, to the affected leg, a torque value calculated by applying an offset angle to a joint angle of the affected leg.

According to an example embodiment, when the right leg is the affected leg, to assist the right leg, the offset angle may be applied to a right hip joint angle. In a right torque trajectory 1110 and a left torque trajectory 1112 output based on the offset angle, a flexion interval 1120 of the right leg may be longer than a flexion interval 1122 of the left leg, and a magnitude of a torque may also increase. As the flexion interval 1120 of the right leg increases, and the magnitude of the torque increases, the right leg, which is the affected leg, may be effectively assisted. A symmetric increase of torques output respectively to the right leg and the left leg may prevent the user from feeling inconvenience from asymmetry of the torque output.

FIG. 12 is a flowchart illustrating a method of determining a first value of a parameter, based on an operation mode of a wearable device according to an embodiment.

According to an example embodiment, operation 540 described above with reference to FIG. 5 may include operations 1210 to 1230 to be described below.

In operation 1210, the processor 142 may determine a target operation mode among one or more operation modes that may be performed by the wearable device 100. The operation modes, for example, may include an assistive mode for assisting a user in walking and a resistive mode for preventing the user from walking. The resistive mode may be an exercise mode. When the user performs an asymmetric gait, the wearable device 100 may provide a resistive force to an affected leg to increase muscular strength of the affected leg.

According to an example embodiment, the user may input a desired operation mode through a UI of the wearable device 100 or an electronic device (e.g., the electronic device 200 of FIG. 2) connected to the wearable device 100.

In operation 1220, the processor 142, when the operation mode is an assistive mode, may determine a first value of a parameter set for the assistive mode. For example, a first value of a gain as the parameter may be determined to be a positive number to assist motion of the user.

According to an example embodiment, when motion of a first leg and a second leg of the user while walking is asymmetric, the wearable device 100 operating in the assistive mode, by adding an offset angle to a joint angle of the affected leg having relatively insufficient muscular strength, may symmetrically correct the motion of the affected leg and the healthy leg of the user. The user, by repeatedly experiencing symmetric motion of the legs with assistance by the wearable device 100, may remember the symmetric motion of the legs. As the user may be able to repeat the remembered symmetric motion of the legs without the assistance by the wearable device 100, walking posture may be effectively corrected.

In operation 1230, the processor 142, when the operation mode is a resistive mode, may determine a first value of a parameter set for the resistive mode. For example, the first value of the gain as the parameter may be determined to be a negative number to hinder the motion of the user.

According to an example embodiment, when the motion of the first leg and the second leg of the user while walking is asymmetric, the wearable device 100 operating in the resistive mode, by adding the offset angle to the joint angle of the affected leg having relatively insufficient muscular strength, may provide the user with the resistive force to increase the muscular strength of the affected leg. By increasing the muscular strength of the affected leg, the user may decrease asymmetric motion of the legs.

FIG. 13 is a diagram illustrating a trajectory of a torque value output in a resistive mode according to an embodiment.

Referring to FIG. 13, a right angular trajectory 1310 of the right hip joint and a left angular trajectory 1312 of the left hip joint and a right torque trajectory 1320 of the right leg and a left torque trajectory 1312 of the left leg are illustrated.

In the graph illustrated in FIG. 13, an interval 1330, in which a resistive force is output to an interval in which the right leg, which is an affected leg, swings, may be larger and longer than an interval 1332, in which the resistive force is output to an interval in which the left leg swings.

The methods according to the above-described examples may be recorded in non-transitory computer-readable media including program instructions to implement various operations of the above-described examples. The media may also include, alone or in combination with the program instructions, data files, data structures, and the like. The program instructions recorded on the media may be those specially designed and constructed for the purposes of example embodiments, or they may be of the kind well-known and available to those having skill in the computer software arts. Examples of non-transitory computer-readable media include magnetic media such as hard disks, floppy disks, and magnetic tape; optical media such as compact-disc read-only memory (CD-ROM) discs or digital versatile discs (DVDs_; magneto-optical media such as optical discs; and hardware devices that are specially configured to store and perform program instructions, such as ROM, random access memory (RAM), flash memory, and the like. Examples of program instructions include both machine code, such as produced by a compiler, and files containing higher-level code that may be executed by the computer using an interpreter. The above-described devices may be configured to act as one or more software modules in order to perform the operations of the above-described example embodiments, or vice versa.

A number of example embodiments have been described above. Nevertheless, it should be understood that various modifications may be made to these example embodiments. Suitable results may be achieved if the described techniques are performed in a different order, and/or if components in a described system, architecture, device, or circuit are combined in a different manner, and/or replaced or supplemented by other components or their equivalents.

Therefore, the scope of the disclosure is defined not by the detailed description, but by the claims, and all variations within the scope of the claims are to be construed as being included in the disclosure.

**Claims**

1. A non-therapeutic method, performed by a wearable device (100, 100-1), of outputting a torque, the method comprising:

    obtaining (510) via at least one sensor (120, 121, 121-1) of the wearable device a first angle ($g\_r$) of a first joint of a first leg of a user of the wearable device (100, 100-1) and a second angle ($q\_l$) of a second joint of a second leg of the user;

    obtaining (520) via a processor (142) of the wearable device a first adjustment angle, based on an offset angle set for the first angle ($g\_r$) and the first joint;

    obtaining (530) via the processor a first state factor associated with the first angle ($g\_r$) and the second angle ($q\_l$), based on the first adjustment angle and the second angle ($q\_l$);

    obtaining (540) via the processor a first value of a parameter for adjusting at least one of a magnitude, a direction, and timing of a torque to be output;

    obtaining (550) via the processor a first torque value, based on the first state factor and the first value of the parameter; and

    controlling (560) a motor driver (110, 110-1, 110-2) of the wearable device (100, 100-1) to output the first torque value via a motor (114, 114-1) of the wearable device,

    wherein a maximum value of the first torque value increases as the offset angle increases,

    wherein a flexion interval (1120) of the first joint increases as the offset angle increases, and

    wherein a flexion interval (1122) of the second joint decreases as the offset angle increases.

2. The non-therapeutic method of claim 1, wherein the offset angle is set by the user.

3. The non-therapeutic method of claim 1, further comprising:
    obtaining, via the processor, the offset angle, based on a first angular trajectory (910, 1010) of the first angle ($g\_r$) of the first joint and a second angular trajectory (912, 1012) of the second angle ($q\_l$) of the second joint.

4. The non-therapeutic method of claim 3, wherein the obtaining the offset angle comprises:
    obtaining (710), via the processor, a target asymmetry degree, based on the first angular trajectory (910, 1010) and the second angular trajectory (912, 1012).

5. The non-therapeutic method of claim 1, wherein the first state factor is associated with a distance between the first leg and the second leg.

6. The non-therapeutic method of claim 1, wherein the obtaining (530) the first state factor comprises:

    obtaining, via the processor, an initial state factor, based on the first adjustment angle and the second angle ($q\_l$), and

    wherein the first state factor is determined via the processor based on a previous state factor and the initial state factor.

7. The non-therapeutic method of claim 1, wherein the parameter comprises at least one of a gain and a delay associated with the first state factor.

8. The non-therapeutic method of claim 7, wherein a first value of the gain and a first value of the delay are set differently based on a target operation (510, 540, 700, 1210) mode among one or more operation (510, 540, 700, 1210) modes of the wearable device (100, 100-1).

9. The non-therapeutic method of claim 8, wherein the one or more operation (510, 540, 700, 1210) modes comprise: an assistive mode for outputting the first torque value in a first direction that is the same as a second direction in which the first joint moves, and a resistive mode for outputting the first torque value in a third direction opposite to the second direction in which the first joint moves.

10. The non-therapeutic method of claim 1, wherein the first value of the parameter is determined differently based on the offset angle.

11. A non-transitory computer-readable storage medium storing instructions that, when executed by a processor (142) of a wearable device (100, 100-1), cause the processor (142) to perform the non-therapeutic method of claim 1.

12. A wearable device (100-1) configured to provide a force to a user, the wearable device (100-1) comprising:

at least one sensor (120, 121, 121-1) configured to measure an angle of a joint of the user;
a motor driver circuit (112, 112-1);
a motor (114, 114-1) electrically connected to the motor driver circuit (112, 112-1); and
a processor (142) configured to:

obtain (510), by the at least one sensor (120, 121, 121-1), a first angle ($g\_r$) of a first joint of a first leg of the user and a second angle ($q\_l$) of a second joint of a second leg of the user,
obtain (520) a first adjustment angle, based on an offset angle set for the first angle ($g\_r$) and the first joint,
obtain (530) a first state factor associated with the first angle ($g\_r$) and the second angle ($q\_l$), based on the first adjustment angle and the second angle ($q\_l$),
obtain (540) a first value of a parameter for adjusting at least one of a magnitude, a direction, and timing of a torque to be output,
obtain (550) a first torque value, based on the first state factor and the first value of the parameter, and
control (560) the motor driver circuit (112, 112-1) to output the first torque value,
wherein a maximum value of the first torque value increases as the set offset angle increases,
wherein the force provided to the user corresponds the output first torque value,
wherein a flexion interval (1120, 1122) of the first joint increases as the offset angle increases, and
wherein a flexion interval (1120, 1122) of the second joint decreases as the offset angle increases.

13. The wearable device (100-1) of claim 12, wherein the processor (142) is further configured to:

obtain (710) a target asymmetry degree, based on a first angular trajectory (910, 1010) of the first angle ($g\_r$) of the first joint and a second angular trajectory (912, 1012) of the second angle ($q\_l$) of the second joint, and
obtain the offset angle based on the target asymmetry degree.

**Patentansprüche**

1. Nicht-therapeutisches Verfahren zum Ausgeben eines Drehmoments, das von einem tragbaren Gerät (100, 100-1) durchgeführt wird, wobei das Verfahren Folgendes umfasst:

Ermitteln (510) eines ersten Winkels ($g\_r$) eines ersten Gelenks eines ersten Beins eines Benutzers des tragbaren Geräts (100, 100-1) und eines zweiten Winkels ($q\_l$) eines zweiten Gelenks eines zweiten Beins des Benutzers über mindestens einen Sensor (120, 121, 121-1) des tragbaren Geräts;
Ermitteln (520) eines ersten Einstellwinkels über einen Prozessor (142) des tragbaren Geräts, basierend auf einem für den ersten Winkel ($g\_r$) und das erste Gelenk festgelegten Versatzwinkel;
Ermitteln (530) eines ersten Zustandsfaktors, der mit dem ersten Winkel ($g\_r$) und dem zweiten Winkel ($q\_1$) assoziiert ist, über den Prozessor basierend auf dem ersten Einstellwinkel und dem zweiten Winkel ($q\_l$);
Ermitteln (540) eines ersten Werts eines Parameters zum Einstellen mindestens einer bzw. eines von einer Magnitude, einer Richtung und einem Zeitpunkt, zu dem ein Drehmoment ausgegeben wird, über den Prozessor;
Ermitteln (550) eines ersten Drehmomentwerts über den Prozessor auf der Grundlage des ersten Zustandsfaktors und des ersten Werts des Parameters; und
Steuern (560) eines Motortreibers (110, 110-1, 110-2) des tragbaren Geräts (100, 100-1), um den ersten Drehmomentwert über einen Motor (114, 114-1) des tragbaren Geräts auszugeben,
wobei ein Maximalwert des ersten Drehmomentwerts mit zunehmendem Versatzwinkel zunimmt,
wobei ein Flexionsintervall (1120) des ersten Gelenks mit zunehmendem Versatzwinkel zunimmt und
wobei ein Flexionsintervall (1122) des zweiten Gelenks mit zunehmendem Versatzwinkel abnimmt.

2. Nicht-therapeutisches Verfahren nach Anspruch 1, wobei der Versatzwinkel vom Benutzer eingestellt wird.

3. Nicht-therapeutisches Verfahren nach Anspruch 1, umfassend ferner:
Ermitteln des Versatzwinkels über den Prozessor auf der Grundlage einer ersten Winkelbahn (910, 1010) des ersten Winkels ($g\_r$) des ersten Gelenks und einer zweiten Winkelbahn (912, 1012) des zweiten Winkels ($q\_l$) des zweiten Gelenks.

**4.** Nicht-therapeutisches Verfahren nach Anspruch 3, wobei das Ermitteln des Versatzwinkels Folgendes umfasst: Ermitteln (710) eines Zielsymmetriegrades über den Prozessor auf der Grundlage der ersten Winkelbahn (910, 1010) und der zweiten Winkelbahn (912, 1012).

**5.** Nicht-therapeutisches Verfahren nach Anspruch 1, wobei der erste Zustandsfaktor mit einem Abstand zwischen dem ersten Bein und dem zweiten Bein assoziiert ist.

**6.** Nicht-therapeutisches Verfahren nach Anspruch 1, wobei das Ermitteln (530) des ersten Zustandsfaktors Folgendes umfasst:

> Ermitteln eines Anfangszustandsfaktors über den Prozessor auf der Grundlage des ersten Einstellwinkels und des zweiten Winkels (q_l), und
> wobei der erste Zustandsfaktor über den Prozessor auf der Grundlage eines vorherigen Zustandsfaktors und des Anfangszustandsfaktors bestimmt wird.

**7.** Nicht-therapeutisches Verfahren nach Anspruch 1, wobei der Parameter mindestens eine von einer Verstärkung und einer Verzögerung umfasst, die mit dem ersten Zustandsfaktor assoziiert sind.

**8.** Nicht-therapeutisches Verfahren nach Anspruch 7, wobei ein erster Wert der Verstärkung und ein erster Wert der Verzögerung basierend auf einem Zielbetriebsmodus (510, 540, 700, 1210) unter einem oder mehreren Betriebsmodi (510, 540, 700, 1210) des tragbaren Geräts (100, 100-1) unterschiedlich eingestellt werden.

**9.** Nicht-therapeutisches Verfahren nach Anspruch 8, wobei der eine oder die mehreren Betriebsmodi (510, 540, 700, 1210) Folgendes umfassen: einen Unterstützungsmodus zum Ausgeben des ersten Drehmomentwerts in einer ersten Richtung, die mit einer zweiten Richtung übereinstimmt, in der sich das erste Gelenk bewegt, und einen Widerstandsmodus zum Ausgeben des ersten Drehmomentwerts in einer dritten Richtung, die der zweiten Richtung, in der sich das erste Gelenk bewegt, entgegengesetzt ist.

**10.** Nicht-therapeutisches Verfahren nach Anspruch 1, wobei der erste Wert des Parameters auf der Grundlage des Versatzwinkels unterschiedlich bestimmt wird.

**11.** Nicht vorübergehendes, computerlesbares Speichermedium, auf dem Befehle gespeichert sind, die, wenn sie von einem Prozessor (142) eines tragbaren Geräts (100, 100-1) ausgeführt werden, den Prozessor (142) veranlassen, das nicht-therapeutische Verfahren nach Anspruch 1 durchzuführen.

**12.** Tragbares Gerät (100-1), das dazu konfiguriert ist, eine Kraft auf einen Benutzer auszuüben, wobei das tragbare Gerät (100-1) Folgendes umfasst:

> mindestens einen Sensor (120, 121, 121-1), der dazu konfiguriert ist, einen Winkel eines Gelenks des Benutzers zu messen;
> eine Motortreiberschaltung (112, 112-1);
> einen Motor (114, 114-1), der elektrisch mit der Motortreiberschaltung (112, 112-1) verbunden ist; und
> einen Prozessor (142), der dazu konfiguriert ist,
> durch den mindestens einen Sensor (120, 121, 121-1) einen ersten Winkel (g_r) eines ersten Gelenks eines ersten Beins des Benutzers und einen zweiten Winkel (q_l) eines zweiten Gelenks eines zweiten Beins des Benutzers zu ermitteln (510),
> auf der Grundlage eines für den ersten Winkel (g_r) und das erste Gelenk festgelegten Versatzwinkels einen ersten Einstellwinkel zu ermitteln (520),
> auf der Grundlage des ersten Einstellwinkels und des zweiten Winkels (q_l) einen ersten Zustandsfaktor, der mit dem ersten Winkel (g_r) und dem zweiten Winkel (q_l) assoziiert ist, zu ermitteln (530),
> einen ersten Wert eines Parameters zum Einstellen mindestens einer bzw. eines von einer Magnitude, einer Richtung und einem Zeitpunkt, zu dem ein Drehmoment ausgegeben wird, zu ermitteln (540),
> auf der Grundlage des ersten Zustandsfaktors und des ersten Werts des Parameters einen ersten Drehmomentwert zu ermitteln (550) und
> die Motortreiberschaltung (112, 112-1) zu steuern (560), um den ersten Drehmomentwert auszugeben,
> wobei ein Maximalwert des ersten Drehmomentwerts mit dem zunehmenden eingestellten Versatzwinkel zunimmt,

wobei die auf den Benutzer ausgeübte Kraft dem ausgegebenen ersten Drehmomentwert entspricht,
wobei ein Flexionsintervall (1120, 1122) des ersten Gelenks mit zunehmendem Versatzwinkel zunimmt, und
wobei ein Flexionsintervall (1120, 1122) des zweiten Gelenks mit zunehmendem Versatzwinkel abnimmt.

**13.** Tragbares Gerät (100-1) nach Anspruch 12, wobei der Prozessor (142) ferner dazu konfiguriert ist,

auf der Grundlage einer ersten Winkelbahn (910, 1010) des ersten Winkels (g_r) des ersten Gelenks und einer zweiten Winkelbahn (912, 1012) des zweiten Winkels (q_l) des zweiten Gelenks einen Zielasymmetriegrad zu ermitteln (710), und
auf der Grundlage des Zielasymmetriegrades den Versatzwinkel zu ermitteln.

**Revendications**

**1.** Procédé non thérapeutique, réalisé par un dispositif pouvant être porté (100, 100-1), de production d'un couple, le procédé comprenant :

obtenir (510) via au moins un capteur (120, 121, 121-1) du dispositif pouvant être porté un premier angle (g_r) d'une première articulation d'une première jambe d'un utilisateur du dispositif pouvant être porté (100, 100-1) et un deuxième angle (q_l) d'une deuxième articulation d'une deuxième jambe de l'utilisateur ;
obtenir (520) via un processeur (142) du dispositif pouvant être porté un premier angle d'ajustement, en se basant sur un angle de décalage défini pour le premier angle (g_r) et la première articulation ;
obtenir (530) via le processeur un premier facteur d'état associé au premier angle (g_r) et au deuxième angle (q_l), en se basant sur le premier angle d'ajustement et le deuxième angle (q_l) ;
obtenir (540) via le processeur une première valeur d'un paramètre pour ajuster au moins un élément parmi l'ampleur, la direction, et le moment d'un couple à produire ;
obtenir (550) via le processeur une première valeur de couple, en se basant sur le premier facteur d'état et la première valeur du paramètre ; et
contrôler (560) une commande de moteur (110, 110-1, 110-2) du dispositif pouvant être porté (100, 100-1) pour produire la première valeur de couple via un moteur (114, 114-1) du dispositif pouvant être porté,
dans lequel la valeur maximum de la première valeur de couple augmente tandis que l'angle de décalage augmente,
dans lequel un intervalle de flexion (1120) de la première articulation augmente tandis que l'angle de décalage augmente, et
dans lequel un intervalle de flexion (1122) de la deuxième articulation diminue tandis que l'angle de décalage diminue.

**2.** Procédé non thérapeutique selon la revendication 1, dans lequel l'angle de décalage est défini par l'utilisateur.

**3.** Procédé non thérapeutique selon la revendication 1, comprenant en outre :
obtenir, via le processeur, l'angle de décalage, en se basant sur une première trajectoire angulaire (910, 1010) du premier angle (g_r) de la première articulation et une deuxième trajectoire angulaire (912, 1012) du deuxième angle (q_l) de la deuxième articulation.

**4.** Procédé non thérapeutique selon la revendication 3, dans lequel obtenir l'angle de décalage comprend :
obtenir (710), via le processeur, un degré d'asymétrie cible, en se basant sur la première trajectoire angulaire (910, 1010) et la deuxième trajectoire angulaire (912, 1012).

**5.** Procédé non thérapeutique selon la revendication 1, dans lequel le premier facteur d'état est associé à une distance entre la première jambe et la deuxième jambe.

**6.** Procédé non thérapeutique selon la revendication 1, dans lequel obtenir (530) le premier facteur d'état comprend :

obtenir, via le processeur, un facteur d'état initial, en se basant sur le premier angle d'ajustement et le deuxième angle (q_l), et
dans lequel le premier facteur d'état est déterminé via le processeur en se basant sur un facteur d'état précédent et le facteur d'état initial.

7. Procédé non thérapeutique selon la revendication 1, dans lequel le paramètre comprend au moins un élément parmi un gain et un retard associés au premier facteur d'état.

8. Procédé non thérapeutique selon la revendication 7, dans lequel une première valeur du gain et une première valeur du retard sont définies différemment en se basant sur un mode d'opération cible (510, 540, 700, 1210) parmi un ou plusieurs modes d'opération (510, 540, 700, 1210) du dispositif pouvant être porté (100, 100-1).

9. Procédé non thérapeutique selon la revendication 8, dans lequel le un ou plusieurs modes d'opération (510, 540, 700, 1210) comprend :
un mode d'assistance pour produire la première valeur de couple dans une première direction qui est la même que la deuxième direction dans laquelle la première articulation se déplace, et un mode résistif pour produire la première valeur de couple dans une troisième direction opposée à la deuxième direction dans laquelle la première articulation se déplace.

10. Procédé non thérapeutique selon la revendication 1, dans lequel la première valeur du paramètre est déterminée différemment en se basant sur l'angle de décalage.

11. Support de stockage non-transitoire lisible par ordinateur stockant des instructions qui, lorsqu'exécutées par un processeur (142) d'un dispositif pouvant être porté (100, 100-1), amènent le processeur (142) à réaliser le procédé non thérapeutique selon la revendication 1.

12. Dispositif pouvant être porté (100-1) configuré pour fournir une force à un utilisateur, le dispositif pouvant être porté (100-1) comprenant :

au moins un capteur (120, 121, 121-1) configuré pour mesurer un angle d'une articulation de l'utilisateur ;
un circuit de commande de moteur (112, 112-1) ;
un moteur (114, 114-1) raccordé électriquement au circuit de commande de moteur (112, 112-1) ; et
un processeur (142) configuré pour :

obtenir (510) via le au moins un capteur (120, 121, 121-1) un premier angle (g_r) d'une première articulation d'une première jambe d'un utilisateur et un deuxième angle (q_l) d'une deuxième articulation d'une deuxième jambe de l'utilisateur,
obtenir (520) un premier angle d'ajustement, en se basant sur un angle de décalage défini pour le premier angle (g_r) et la première articulation,
obtenir (530) un premier facteur d'état associé au premier angle (g_r) et au deuxième angle (q_l), en se basant sur le premier angle d'ajustement et le deuxième angle (q_l),
obtenir (540) une première valeur d'un paramètre pour ajuster au moins un élément parmi l'ampleur, la direction, et le moment d'un couple à produire ;
obtenir (550) une première valeur de couple, en se basant sur le premier facteur d'état et la première valeur du paramètre ; et
contrôler (560) une commande de moteur (112, 112-1) pour produire la première valeur de couple,
dans lequel la valeur maximum de la première valeur de couple augmente tandis que l'angle de décalage augmente,
dans lequel la force fournie à l'utilisateur correspond à la première valeur de couple produire,
dans lequel un intervalle de flexion (1120, 1122) de la première articulation augmente tandis que l'angle de décalage augmente, et
dans lequel un intervalle de flexion (1120, 1122) de la deuxième articulation diminue tandis que l'angle de décalage diminue.

13. Un dispositif pouvant être porté (100-1) selon la revendication 12, dans lequel le processeur (142) est en outre configuré pour :

obtenir (710) un degré d'asymétrie cible, en se basant sur la première trajectoire angulaire (910, 1010) du premier angle (g_r) de la première articulation et une deuxième trajectoire angulaire (912, 1012) du deuxième angle (q_l) de la deuxième articulation, et
obtenir un angle de décalage basé sur le degré d'asymétrie cible.

FIG. 1A

FIG. 1B

FIG. 1C

FIG. 1D

FIG. 2

110-2   110-1

100

z

x

FIG. 3

FIG. 4

Start

Obtain first angle of first joint of first leg of user and
second angle of second joint of second leg of user — 510

Calculate first adjustment angle, based on first angle and offset angle — 520

Determine first state factor, based on first adjustment angle
and second angle — 530

Determine first value of parameter associated with first state factor — 540

Determine first torque value, based on first state factor
and first value of parameter — 550

Control motor driver to output first torque value — 560

End

FIG. 5

**FIG. 6**

From operation 510

700

710

Determine target asymmetry degree, based on first angular trajectory of angle of first joint and second angular trajectory of angle of second joint

720

Determine offset angle corresponding to target asymmetry degree

To operation 520

**FIG. 7**

FIG. 8A

FIG. 8B

FIG. 9

FIG. 10

FIG. 11

From operation 530

540

Determine operation mode
1210

Operate in assistive mode
1220

Operate in resistive mode
1230

To operation 550

FIG. 12

FIG. 13

**EP 4 335 427 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 3037081 A1
- US 10350129 B2
- US 2012215140 A1
- US 9610209 B2
- US 2015366738 A1